(19) Europäisches Patentamt

European Patent Office

Office européen des brevets

(11) **EP 1 731 620 A1**

(12) **EUROPEAN PATENT APPLICATION**

(43) Date of publication:
**13.12.2006 Bulletin 2006/50**

(51) Int Cl.:
***C12Q 1/68*** (2006.01)

(21) Application number: **05291215.1**

(22) Date of filing: **07.06.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(71) Applicant: **INSTITUT NATIONAL DE LA SANTE ET
DE LA RECHERCHE MEDICALE (INSERM)
75013 Paris (FR)**

(72) Inventors:
• **Brouard, Sophie
44240 Suce sur Erdre (FR)**

• **Soulillou, Jean-Paul
44100 Nantes (FR)**
• **Baeten, Dominique
9070 Destelbergen (BE)**
• **Braud, Christophe
44000 Nantes (FR)**
• **Giral, Magali
44470 Carquefou (FR)**

(74) Representative: **Warcoin, Jacques et al
Cabinet Régimbeau
20, rue de Chazelles
75847 Paris cedex 17 (FR)**

(54) **Method for the diagnosis of immune graft tolerance**

(57)    The present invention concerns a method for the in vitro diagnosis of a graft tolerant phenotype, comprising : determining from a grafted subject biological sample an expression profile comprising at least one gene identified in the present invention as differentially expressed between graft tolerant and graft non-tolerant subjects, optionally measuring other parameters, and determining the presence or absence of a graft tolerant phenotype from said expression profile and optional other parameters. The invention further concerns kits and oligonucleotide microarrays suitable to implement said method.

EP 1 731 620 A1

**Description**

The present invention concerns a method for the in vitro diagnosis of a graft tolerant phenotype, comprising : determining from a grafted subject biological sample an expression profile comprising at least one gene identified in the present invention as differentially expressed between graft tolerant and graft non-tolerant subjects, optionally measuring other parameters, and determining the presence or absence of a graft tolerant phenotype from said expression profile and optional other parameters. The invention further concerns kits and oligonucleotide microarrays suitable to implement said method.

Currently, the long-term survival of an allograft is depending on the continuous administration of immunosuppressive drugs. Indeed, an interruption of the immunosuppressive treatment generally leads to an acute or chronic rejection, particularly in case of an early or abrupt diminution (Dantal J. et al. 1998. Lancet. 351:623-8).

However, long-term immunosuppressive treatments lead to severe side effects such as chronic nephrotoxicity, an increased susceptibility to opportunistic infections, and a dose-dependant increased propensity to develop cancers (Iman A. et al. 2001. Natl. Med. J. India. 14(2):75-80).

Despite the difficulties encountered by many attempts to induce a persistent tolerance to allografts in human, it has been observed that some patients can maintain the tolerance to their graft without any immunosuppressive treatment (Brouard S. et al. 2005. Am. J. Transplant. 5(2): 330-340), demonstrating that a tolerant state may naturally occur, even in humans.

In the case of kidney graft, the real proportion of tolerant grafted subjects may be underestimated. Indeed, although the possibility to progressively stop the immunosuppressive treatment has never been investigated, a significant proportion of kidney grafted subject tolerate their graft with a minimal dose of immunosuppressive drug (cortisone monotherapy, <10mg a day) (Brouard S. et al. 2005. Am. J. Transplant. 5(2): 330-340).In addition, among patients developing post-transplantation lymphoproliferative disorders, leading to the interruption of their immunosuppressive treatment, a significant proportion do not reject their graft.

Thus, a significant proportion of kidney grafted subjects might display an unsuspected, total or partial, immune tolerance state to their graft. It would therefore be very useful to have a method to diagnose, without any previous modification of the immunosuppressive treatment, the level of immune tolerance of grafted subjects to their graft. Indeed, this would allow for an ethically acceptable, progressive, total or partial withdrawal of immunosuppressive drugs in subject with a high enough level of graft tolerance. Although well known biological parameters are used by clinicians for the evaluation of renal function (creatinine and urea serum concentrations and clearance), these parameters are not sufficient for a precise diagnosis of tolerance or rejection. Currently, only a biopsy of the grafted kidney allows, through the analysis of the presence or absence of several histological lesions types (Nankivell et al. 2003. N Engl J Med. 349(24):2326-33), for the precise evaluation of said grafted kidney functionality. A biopsy is an invasive examination, which is not without danger for the grafted organ, and is thus usually not performed on grafted subjects that have stable biological parameters values. A non-invasive method of precise diagnosis of a graft tolerant phenotype is thus needed.

In addition, in the case of many grafted organ, when the values of standard biological parameters allow for the diagnostic of chronic rejection, the rejection process is already in progress and, although it may in certain cases be stopped, the lesions that have been induced generally cannot be reversed. Moreover, to confirm the diagnostic, a biopsy of the grafted organ is usually performed, which is, as stated before, not without danger. It would thus also be very valuable to have a non-invasive method allowing to diagnose chronic rejection at the earlier steps of the rejection process, which would permit to adapt the immunosuppressive treatment and might in some cases prevent the chronic rejection process.

Finally, a non-invasive method for an early and reliable diagnosis of a graft tolerant or non-tolerant phenotype would be very useful in clinical research, since it would allow for relatively short (6 months to 1 year), and thus less expensive, clinical trial studies.

At the present time, few genome-wide studies have been carried out in humans on the modifications of gene expression patterns after kidney transplant. In addition, these studies focused on the identification of genes implicated in graft acute or chronic rejection, and not in graft tolerance. From the comparison of the expression level of about 40 000 genes in tolerant patients versus patients in chronic rejection, the inventors identified a few hundred genes that are significantly differentially expressed between the two groups of patients.

Thanks to identification of these genes that are significantly differentially expressed between tolerant patients and patients in chronic rejection, it is now possible to use a non-invasive method of in vitro diagnosis of a graft tolerant or, on the contrary, a graft non-tolerant phenotype. Such a method allows for the identification of grafted subject for whom a progressive, total or partial withdrawal of immunosuppressive drugs is possible. It also permits an early diagnosis of a chronic rejection process in patients whose biological parameters levels are still normal. Moreover, the diagnosis may be performed from a blood sample, which is completely harmless for the tested grafted subject.

The invention thus concerns a method for the in vitro diagnosis of a graft tolerant phenotype, comprising :

(a) determining from a grafted subject biological sample an expression profile comprising at least one gene from Table 1, and

(b) determining the presence or absence of a graft tolerant phenotype from said expression profile.

According to the present invention, a "graft tolerant phenotype" is defined as a state of tolerance of a subject to his graft. A "state of tolerance" means that this subject does not reject his graft in the absence of an immunosuppressive treatment. As used herein, a "graft tolerant" subject is thus a subject displaying a graft tolerant phenotype, while a subject that do not display a graft tolerant phenotype will be referred to as a "graft non-tolerant" subject.

Immunosuppressive drugs that may be employed in transplantation procedures include azathioprine, methotrexate, cyclophosphamide, FK-506, rapamycin, corticosteroids, and cyclosporins. These drugs may be used in monotherapy or in combination therapies.

In the case of kidney graft, the following immunosuppressive protocols are usually used.

Subjects with primary kidney graft generally receive an induction treatment consisting of 2 injections of basiliximab (Simulect®, a chimeric murine/human monoclonal anti IL2-Rα, antibody commercialized by Novartis), in association with tacrolimus (Prograf™, Fujisawa Pharmaceutical, 0.1 mg/kg/day), mycophenolate mofetil (Cellcept™, Syntex Laboratories, Inc, 2 g/day) and corticoids (1 mg/kg/day), the corticoid treatment being progressively decreased of 10 mg every 5 days until end of treatment, 3 months post transplantation.

Subjects with secondary or tertiary kidney graft, or subjects considered as exposed (percentage of anti-T PRA previously peaking above 25% or cold ischemia for more than 36 hours), generally receive a short anti-thymocyte globulin (ATG) cure (7 days), in association from day 0 with mycophenolate mofetil (Cellcept™, Syntex Laboratories, Inc, 2 g/day)), and corticoids (1 mg/kg/day), the corticoid treatment being progressively decreased of 10 mg every 5 days until end of treatment, 3 months post transplantation, tacrolimus (Prograf™, Fujisawa Pharmaceutical) being introduced in a delayed manner at a dose of 0.1 mg/kg/day.

A "biological sample" may be any sample that may be taken from a grafted subject, such as a serum sample, a plasma sample, a urine sample, a blood sample, a lymph sample, or a biopsy. For the determination of an expression profile, such a sample must allow for the determination of an expression profile comprising at least one gene from Table 1. Preferred biological samples for the determination of an expression profile include samples comprising lymphocytes, such as a blood sample, a lymph sample, or a biopsy. Preferably, the biological sample is a blood sample, more preferably a peripheral blood sample comprising peripheral blood lymphocytes (PBLs). Indeed, such a blood sample may be obtained by a completely harmless blood collection from the grafted patient and thus allows for a non-invasive diagnosis of a graft tolerant or non-tolerant phenotype.

By "expression profile" is meant a group of at least one value corresponding to the expression level of at least one gene. Preferably, the expression profile comprises at least 1, at least 2, at least 3, at least 4, at least 5, at least 8, at least 10, at least 15, at least 20, at least 30, at least 40, at least 50, at least 75, at least 100, at least 200, at least 300, or all genes from Table 1.

272 genes that were determined by the inventors to display significantly different expression levels between kidney graft tolerant subjects as defined above (Tol) and kidney transplanted subjects in chronic rejection (CR) are listed in the following Table 1.

Table 1. 272 genes differentially expressed between kidney transplanted subjects that are tolerant (Tol) or in chronic rejection (CR).

| Gene accession number (GenBank) and description | Oligonucleotide name | Expression ratio Tol/CR |
|---|---|---|
| gi:18254505 - h2a histone family, member i; h2afi | mwghum40K-B42917 | -7,34619331 |
| gi:20147646 - guanine nucleotide binding protein gamma 10; gng10 | mwghum40K-A40402 | -6,37805874 |
| gi:7688676 - mitochondrial solute carrier | mwghum40K-A02509 | -6,14454261 |
| gi:22063962 - hypothetical protein xp_173518; loc254703 | mwghum40K-B20717 | -5,70262156 |
| gi:5031976 - pre-b-cell colony-enhancing factor; pbef | mwghum40K-A18193 | -5,60842525 |
| gi:22770668 - histone h1; hist1h1c | mwghum40K-A01644 | -5,38639464 |
| gi:4757903 - cathelicidin antimicrobial peptide; camp | mwghum40K-A06829 | -5,33547359 |
| gi:19033999 - interleukin-1 receptor type ii; il1r2 | mwghum40K-A01029 | -5,30670924 |
| gi:7416992 - evh1 domain binding protein | mwghum40K-A11152 | -5,05831189 |
| gi:20562106 - hypothetical protein xp_096998; loc146429 | mwghum40K-B18430 | -4,89777252 |

(continued)

| Gene accession number (GenBank) and description | Oligonucleotide name | Expression ratio Tol/CR |
|---|---|---|
| gi:10835138 - fc fragment of igg, low affinity iiib, receptor for (cd16); fcgr3b | mwghum40K-A08228 | -4,87344152 |
| gi:5031852 - lipocalin 2 (oncogene 24p3); lcn2 | mwghum40K-A00548 | -4,83671046 |
| gi:20553683 - hypothetical protein xp_069436; loc135570 | mwghum40K-B23564 | -4,73529079 |
| gi:338285 - superoxide dismutase | mwghum40K-A07525 | -4,70106972 |
| gi:18546082 - similar to netrin g1; loc163474 | mwghum40K-B43065 | -4,68085049 |
| gi:10435550 - cdna clone unnamed protein product. | mwghum40K-A09782 | -4,55814542 |
| gi:20357558 - charot-leyden crystal protein; clc | mwghum40K-A42317 | -4,52312165 |
| gi:18088891 - unknown (protein for mgc:31837) | mwghum40K-B33973 | -4,48003114 |
| gi:21389161 - testis-specific kinase-1 | mwghum40K-A44797 | -4,44263119 |
| gi:5107944 - cd163 | mwghum40K-A13213 | -4,43234759 |
| gi:22043774 - similar to hypothetical protein dj328e19.c1.1; loc257031 | mwghum40K-B42695 | -4,35198868 |
| gi:178854 - apoj | mwghum40K-A07050 | -4,31690852 |
| gi:17865815 - vanin 2, isoform 2; vnn2 | mwghum40K-A03363 | -4,27919914 |
| gi:17385764 - rtp801 | mwghum40K-A13363 | -4,27858794 |
| gi:4235636 - myeloid cell leukemia sequence 1; mcl1 | mwghum40K-A01532 | -4,25975866 |
| gi:15080489 - similar to cd9 antigen (p24) | mwghum40K-A08384 | -4,24008734 |
| gi:9971203 - translocation protein 1; htp1 | mwghum40K-A06017 | -4,21166173 |
| gi:190069 - itga2b | mwghum40K-A08420 | -4,17079447 |
| gi:8051587 - ficolin 2 isoform c precursor; fcn2 | mwghum40K-A40139 | -4,13854201 |
| gi:5174654 - reticulon 3; rtn3 | mwghum40K-A09221 | -4,07343746 |
| gi:21707189 - sp2 transcription factor | mwghum40K-A11502 | -4,03974063 |
| gi:2853300 - mucin; muc3 | mwghum40K-B44204 | -4,01428519 |
| gi:20548888 - similar to evidence:nas-hypothetical protein-putative; loc207038 | mwghum40K-B23396 | -3,97268924 |
| gi:20535212 - hypothetical protein xp_117253; loc200589 | mwghum40K-B24491 | -3,95465948 |
| gi:4377847 - transformer-2-beta isoform 1; sfrs10 | mwghum40K-A30672 | -3,93843939 |
| gi:4885516 - nuclear factor, interleukin 3 regulated; nfil3 | mwghum40K-A05458 | -3,93259777 |
| gi:18572402 - similar to h23l24.3.p; loc158135 | mwghum40K-A29472 | -3,88771054 |
| gi:182666 - fmlp-related receptor ii; fmlp r ii | mwghum40K-A01843 | -3,88520187 |
| gi:4758711 - maltase-glucoamylase; mgam | mwghum40K-A10060 | -3,88410527 |
| gi:17444765 - hypothetical protein xp_065786; loc130582 | mwghum40K-B20413 | -3,87904893 |
| gi:13477170 - unknown (protein for mgc:12852) | mwghum40K-A18616 | -3,82921092 |
| gi:14041914 - cdna clone moderately similar to peptidyl-prolyl cis-trans isomerase 10 (ec 5.2.1.8); unnamed protein product. | mwghum40K-A40845 | -3,81858669 |
| gi:16359142 - serine/threonine kinase 17b (apoptosis-inducing) | mwghum40K-A01274 | -3,81166844 |

(continued)

| Gene accession number (GenBank) and description | Oligonucleotide name | Expression ratio Tol/CR |
|---|---|---|
| gi:21410262 - sorting nexin 10 | mwghum40K-A08040 | -3,79731458 |
| gi:15029599 - hypothetical protein flj22662 | mwghum40K-A09134 | -3,73451226 |
| gi:10437677 - homo sapiens cdna: flj21558 fis, done col06372; unnamed protein product. | mwghum40K-A13460 | -3,71239164 |
| gi:2697102 - heterogeneous nuclear ribonucleoprotein r | mwghum40K-A00281 | -3,69800135 |
| gi:21070975 - second mitochondria-derived activator of caspase, isoform smac-delta, precursor; smac | mwghum40K-A05137 | -3,65972323 |
| gi:4240256 - kiaa0884 protein; kiaa0884 | mwghum40K-A14205 | -3,65150207 |
| gi:18375654 - protein tyrosine phosphatase, non-receptor type 18; ptpn18 | mwghum40K-A01239 | -3,64569258 |
| gi:1813603 - mhc class i antigen; hla-b | mwghum40K-A39868 | -3,62541957 |
| gi:16549826 - cdna clone weakly similar to tlm protein; unnamed protein product. | mwghum40K-B31744 | -3,59505057 |
| gi:19718733 - toll-like receptor 2; tlr2 | mwghum40K-A05073 | -3,57853391 |
| gi:7228287 - hypothetical protein | mwghum40K-A00945 | -3,50015151 |
| gi:12642794 - il-5 promoter reii-region-binding protein; reiibp | mwghum40K-A30298 | -3,47250934 |
| gi:20380066 - similar to kiaa0616 protein | mwghum40K-A04868 | -3,39791863 |
| gi:2773159 - neuronal tissue-enriched acidic protein; nap-22 | mwghum40K-A09698 | -3,34503294 |
| gi:16306617 - phosphatidylserine decarboxylase | mwghum40K-A03405 | -3,33015128 |
| gi:602451 - cytidine deaminase; cda | mwghum40K-A04522 | -3,32160322 |
| gi:4504906 - karyopherin (importin) beta 2; kpnb2 | mwghum40K-A09218 | -3,3123785 |
| gi:15426543 - similar to c-type (calcium dependent, carbohydrate-recognition domain) lectin, superfamily member 12 | mwghum40K-A30974 | -3,28905653 |
| gi:11932151 - cell surface receptor fdfact2; fdfact2 | mwghum40K-A09000 | -3,2628189 |
| gi:9716908 - transient receptor potential channel 6; trp6 | mwghum40K-A42490 | -3,26266826 |
| gi:13162185 - calsyntenin-3 protein | mwghum40K-A16184 | -3,24520672 |
| gi:4455493 - dj28o10.2 (g0s2 (putative lymphocyte g0/g1 switch protein 2)); dj28o10.2 | mwghum40K-A09558 | -3,23443841 |
| gi:21750836 - cdna clone highly similar to homo sapiens acetyl-coa synthetase mrna; unnamed protein product. | mwghum40K-A01510 | -3,21624039 |
| gi:8050526 - triggering receptor expressed on monocytes 1 | mwghum40K-A07532 | -3,18617684 |
| gi:5821287 - macrophage c-type lectin mincle; mincle | mwghum40K-A11131 | -3,16332929 |
| gi:16307110 - unknown (protein for mgc: 17869) | mwghum40K-A30708 | -3,14463754 |
| gi:15987820 - coagulation factor xiii, a1 polypeptide; f13a1 | mwghum40K-A06479 | -3,13812972 |
| gi:11139019 - eosinophil-derived neurotoxin | mwghum40K-A09312 | -3,13762597 |
| gi:13549115 - platelet factor 4 | mwghum40K-A03620 | -3,126771 |
| gi:8489812 - dj963k23.2 | mwghum40K-A08293 | -3,11784668 |

(continued)

| Gene accession number (GenBank) and description | Oligonucleotide name | Expression ratio Tol/CR |
|---|---|---|
| gi:10435179 - cdna clone unnamed protein product. | mwghum40K-A01363 | -3,0861361 |
| gi:16550539 - cdna Gone unnamed protein product. | mwghum40K-A29707 | -3,08078215 |
| gi:21536278 - cartilage associated protein; crtap | mwghum40K-A17689 | -3,07800073 |
| gi:20543600 - similar to ph (pleckstrin homology) domain; kiaa1200 | mwghum40K-A14595 | -3,07074188 |
| gi:9800411 - thromboxane synthase; cyp5a1 | mwghum40K-A01891 | -3,06824551 |
| gi:7021945 - cdna Gone weakly similar to ovarian granulosa cell 13.0 kd protein hgr74; unnamed protein product. | mwghum40K-A17113 | -3,04491282 |
| gi:20072212 - lymphotoxin beta receptor (tnfr superfamily, member 3) | mwghum40K-A09774 | -3,03599846 |
| gi:9931332 - cysteine-rich secreted protein; fizz3 | mwghum40K-A19615 | -2,97938101 |
| gi:13097326 - nucleolar protein family a, member 1 (h/aca small nucleolar mps) | mwghum40K-A04407 | -2,97662457 |
| gi:18307850 - angiogenin | mwghum40K-A32777 | -2,96587185 |
| gi:190137 - p78-related protein | mwghum40K-A03097 | -2,9636468 |
| gi:187270 - lyn b protein | mwghum40K-A30109 | -2,95868451 |
| gi:189183 - n-formylpeptide receptor fmlp-r98 | mwghum40K-A00299 | -2,93877547 |
| gi:19879837 - mcef protein; mcef | mwghum40K-A33695 | -2,93315213 |
| gi:5901877 - chondrosarcoma-associated protein 2; csa2 | mwghum40K-A36518 | -2,92495943 |
| gi:4585274 - b-cell activating factor; baff | mwghum40K-A30859 | -2,92257054 |
| gi:4106060 - ccr4-associated factor 1; pop2 | mwghum40K-A18843 | -2,90452189 |
| gi:3859848 - lim protein slimmer | mwghum40K-A19361 | -2,89704729 |
| gi:7022609 - cdna clone unnamed protein product. | mwghum40K-A07056 | -2,89015452 |
| gi:12382236 - transcriptional regulator tsc-22 | mwghum40K-A05288 | -2,87348049 |
| gi:13345797 - adaptor protein p47phox; ncf1 | mwghum40K-A02439 | -2,86285553 |
| gi:12803174 - membrane protein, palmitoylated 1 (55kd) | mwghum40K-A05032 | -2,85763897 |
| gi:18089066 - arginase, liver | mwghum40K-A00480 | -2,85204446 |
| gi:14861637 - putative breast epithelial stromal interaction protein | mwghum40K-A15297 | -2,85031118 |
| gi:10442005 - cervical cancer suppressor-1 | mwghum40K-A29829 | -2,8357473 |
| gi:12653252 - dkfzp586a011 protein | mwghum40K-A08617 | -2,82939777 |
| gi:3600027 - dysferlin | mwghum40K-A35690 | -2,82282316 |
| gi:13543406 - integrin, beta 2 (antigen cd18 (p95), lymphocyte function-associated antigen 1; macrophage antigen 1 (mac-1) beta subunit) | mwghum40K-A32861 | -2,74679548 |
| gi:12654106 - tissue inhibitor of metalloproteinase 1 (erythroid potentiating activity, collagenase inhibitor) | mwghum40K-A42205 | -2,64638406 |
| gi:18645166 - annexin a2 | mwghum40K-A05270 | -2,54972673 |
| gi:183046 - granulocyte colony-stimulating factor receptor; g-csfr-1 | mwghum40K-A05233 | -2,53535967 |

(continued)

| Gene accession number (GenBank) and description | Oligonucleotide name | Expression ratio Tol/CR |
|---|---|---|
| gi:1930109 - gm-csf receptor alpha subunit soluble 3 | mwghum40K-A13645 | -2,45694123 |
| gi:183361 - gm-csf receptor | mwghum40K-A36095 | -2,35100302 |
| gi:4557434 - cd68 antigen; cd68 | mwghum40K-A09540 | -2,34583463 |
| gi:22385326 - vascular cell adhesion molecule 1; vcam1 | mwghum40K-A37320 | -2,32622141 |
| gi:337956 - cell surface glycoprotein cd44; cd44 | mwghum40K-A09385 | -2,21393628 |
| gi:7209680 - il-8 receptor type a; cxcr1 | mwghum40K-A09387 | -2,17434924 |
| gi:179984 - c-c chemokine receptor type 1; cmkr-1 | mwghum40K-A02199 | -2,11813195 |
| gi:22859017 - colony stimulating factor 3 receptor (granulocyte); csf3r | mwghum40K-A38743 | -2,11481516 |
| gi:3983126 - monocyte antigen cd14 precursor; cd14 | mwghum40K-A09621 | -2,07829431 |
| gi:695404 - endothelin converting enzyme | mwghum40K-A04611 | -2,07650215 |
| gi:22212921 - interleukin 11 receptor, alpha isoform 2 precursor; il11ra | mwghum40K-A05133 | -2,06876527 |
| gi:220124 - tissue inhibitor of metalloproteinases; timp | mwghum40K-A44650 | -1,99360911 |
| gi:2997691 - monocyte/neutrophil elastase inhibitor | mwghum40K-A02701 | -1,98300329 |
| gi:22064764 - similar to metalloproteinase inhibitor 2 precursor (timp-2) (tissue inhibitor of metalloproteinases-2) (csc-21k); timp2 | mwghum40K-A19664 | -1,92739859 |
| gi:16357493 - cell division cycle 2-like 2, isoform 9; cdc212 | mwghum40K-A39960 | -1,89660351 |
| gi:18780276 - mucosal vascular addressin cell adhesion molecule 1, isoform c precursor; madcam1 | mwghum40K-A08098 | -1,78687032 |
| gi:21708066 - fc fragment of igg, low affinity iiia, receptor for (cd16) | mwghum40K-A31452 | -1,77857519 |
| gi:17978490 - cd97 antigen, isoform 1 precursor; cd97 | mwghum40K-A04395 | -1,75633171 |
| gi:190697 - platelet activating factor receptor; ptafr | mwghum40K-A06044 | -1,66512964 |
| gi:7328555 - tumor necrosis factor-related death ligand-1 beta | mwghum40K-A18182 | -1,58165806 |
| gi:9800401 - thromboxane synthase; cyp5a1 | mwghum40K-A38514 | -1,55959046 |
| gi:20631963 - bcl2-associated x protein, isoform delta; bax | mwghum40K-A07688 | -1,55153448 |
| gi:22065226 - similar to prothrombin precursor (coagulation factor ii); f2 | mwghum40K-A05450 | -1,49433773 |
| gi:17644180 - tumor necrosis factor, alpha-induced protein 1 (endothelial); tnfaip1 | mwahum40K-A00708 | -1,39068284 |
| gi:15451909 - caspase 4 isoform gamma precursor; casp4 | mwghum40K-A42766 | -1,38613785 |
| gi:15431326 - caspase 1, isoform beta precursor; casp1 | mwghum40K-A10310 | -1,3766428 |
| gi:5031782 - interferon gamma receptor 2 (interferon gamma transducer 1); ifngr2 | mwghum40K-A01285 | -1,32804567 |
| gi:2738962 - natural killer cell receptor kir2dl1 variant | mwghum40K-A41 001 | -1,32776744 |
| gi:180160 - fc gamma receptor type i; cd64 | mwghum40K-A07364 | -1,31431665 |

(continued)

| Gene accession number (GenBank) and description | Oligonucleotide name | Expression ratio Tol/CR |
|---|---|---|
| gi:291928 - cytotoxic t-lymphocyte-associated protein 4; ctla4 | mwghum40K-A04633 | -1,2960322 |
| gi:12803600 - colony stimulating factor 2 receptor, alpha, low-affinity (granulocyte-macrophage) | mwghum40K-A12102 | -1,24696649 |
| gi:3805800 - tumor necrosis factor; tnf | mwghum40K-A09167 | -1,22523078 |
| gi:460284 - granulocyte-macrophage colony-stimulating factor receptor alpha-subunit 3; gm-csf-ra3 | mwghum40K-A14651 | -1,20550336 |
| gi:22477183 - fc fragment of igg, low affinity iiia, receptor for (cd16) | mwghum40K-A37270 | -1,18389815 |
| gi:995296 - interferon receptor; ifnar2 | mwghum40K-A35085 | 1,166813583 |
| gi:5468516 - transforming growth factor-beta 3; tgf-beta 3 | mwghum40K-A17844 | 1,207126881 |
| gi:7108353 - killer cell lectin-like receptor subfamily c, member 2; klrc2 | mwghum40K-A08751 | 1,267394672 |
| gi:19343590 - granzyme m (lymphocyte met-ase 1) | mwghum40K-A03210 | 1,311283254 |
| gi:15081170 - nkg2f | mwghum40K-A03790 | 1,350437408 |
| gi:16507951 - caspase recruitment domain family, member 11; card11 | mwghum40K-A12384 | 1,362678329 |
| gi:16151842 - killer cell lectin-like receptor klrf1-s3; klrf1 | mwghum40K-A35641 | 1,363260921 |
| gi:16605509 - lag-3-associated protein; lap | mwghum40K-A18554 | 1,416308656 |
| gi:12957056 - putative cd28 protein; cd28 | mwghum40K-A40816 | 1,434292584 |
| gi:2459629 - toll-like receptor 5; tlr5 | mwghum40K-A27073 | 1,507514305 |
| gi:12653718 - cdw52 antigen (campath-1 antigen) | mwghum40K-A07615 | 1,515288465 |
| gi:4337095 - lymphotoxin beta | mwghum40K-A01003 | 1,519788833 |
| gi:21636977 - killer cell lectin-like receptor subfamily d transcript 3; klrd1 | mwghum40K-A34506 | 1,723084475 |
| gi:11038675 - cd79b antigen, isoform 2 precursor; cd79b | mwghum40K-A03690 | 1,797423394 |
| gi:12803370 - programmed cell death 10 | mwghum40K-A09821 | 1,813482256 |
| gi:1572492 - bcl-w; bcl-w | mwghum40K-A04820 | 1,880427888 |
| gi:2739159 - interleukin 15 precursor; il-15 | mwghum40K-A00518 | 1,895945637 |
| gi:19923222 - nuclear factor of kappa light polypeptide gene enhancer in b-cells 2 (p49/p100); nfkb2 | mwghum40K-A02845 | 1,939295172 |
| gi:13937862 - cd69 antigen (p60, early t-cell activation antigen) | mwghum40K-A01857 | 2,098520535 |
| gi:18041616 - immunoglobulin kappa light chain variable region; igkv | mwghum40K-B43301 | 2,29182398 |
| gi:13447752 - toll-like receptor 10 | mwghum40K-A11038 | 2,339457716 |
| gi:4504878 - killer cell lectin-like receptor subfamily b, member 1; klrb1 | mwghum40K-A07411 | 2,393013164 |
| gi:558845 - bcl2/adenovirus e1b 19kd-interacting protein 3; bnip3 | mwghum40K-A00806 | 2,474079981 |
| gi:18041573 - immunoglobulin kappa light chain variable region; igkv | mwghum40K-B39608 | 2,786973505 |

(continued)

| Gene accession number (GenBank) and description | Oligonucleotide name | Expression ratio Tol/CR |
|---|---|---|
| gi:18599042 - similar to immunoglobulin-binding protein 1 (cd79a-binding protein 1) (b cell signal transduction molecule alpha 4) (alpha 4 protein); loc166496 | mwghum40K-B42015 | 2,838250508 |
| gi:7676183 - programmed cell death 9; pdcd9 | mwghum40K-A04983 | 2,969303197 |
| gi:15420353 - apoptosis-inhibitor-like protein | mwghum40K-A03576 | 2,978997658 |
| gi:21310431 - immunoglobulin lambda light chain variable region | mwghum40K-B40153 | 2,995905351 |
| gi:4504648 - interleukin 15 receptor, alpha; il15ra | mwghum40K-A07881 | 3,001258197 |
| gi:5578829 - immunoglobulin lambda chain variable region; igvl | mwghum40K-B41742 | 3,034611219 |
| gi:15718705 - caspase 8, isoform b; casp8 | mwghum40K-A08601 | 3,054583366 |
| gi:9857758 - recombinant igg4 heavy chain | mwghum40K-B32278 | 3,20820492 |
| gi:20384707 - annexin ii receptor | mwghum40K-B38395 | 3,351519545 |
| gi:12655588 - immunoglobulin lambda chain variable region; iglv | mwghum40K-B42753 | 3,478053698 |
| gi:189225 - nk4 | mwghum40K-A00277 | 3,52504001 |
| gi:22043624 - similar to evidence:nas~hypothetical protein-putative; loc132299 | mwghum40K-B36305 | 3,526292296 |
| gi:19697921 - trap/mediator complex component trap25; trap25 | mwghum40K-A15241 | 3,527611758 |
| gi:16116858 - immunoglobulin kappa chain variable region; igvko12/2 | mwghum40K-B40559 | 3,534708582 |
| gi:18027739 - unknown; pp12301 | mwghum40K-B13833 | 3,542569092 |
| gi:16549226 - cdna clone weakly similar to polyhomeotic-proximal chromatin protein; unnamed protein product. | mwghum40K-B35913 | 3,545191993 |
| gi:16878208 - unknown (protein for mgc:29715) | mwghum40K-A00437 | 3,589893613 |
| gi:21749768 - cdna clone highly similar to human autoantigen pericentriol material 1 (pcm-1) mrna; unnamed protein product. | mwghum40K-A02285 | 3,593998105 |
| gi:595826 - rgr | mwghum40K-A18095 | 3.615475837 |
| gi:186118 - immunoglobulin lambda light chain c2 region; iglc2 | mwghum40K-B39678 | 3,625138112 |
| gi:2580587 - platelet activating receptor homolog; h963 | mwghum40K-A00078 | 3,641223421 |
| gi:6996445 - chaperonin 10, hsp10 protein; p10 | mwghum40K-A41392 | 3,641225662 |
| gi:22040848 - similar to c-terminal binding protein 2; loc253481 | mwghum40K-B20790 | 3,654507791 |
| gi:2662150 - kiaa0435 | mwghum40K-A05243 | 3,664146492 |
| gi:22044655 - similar to 60s ribosomal protein 17a (surfeit locus protein 3); loc145240 | mwghum40K-A42590 | 3,675842839 |
| gi:10834661 - pp3476 | mwghum40K-B31414 | 3,679136165 |
| gi:537529 - chromosomal protein | mwghum40K-B09006 | 3,685655965 |
| gi:1353823 - ig light chain variable region | mwghum40K-B39861 | 3,697744678 |

(continued)

| Gene accession number (GenBank) and description | Oligonucleotide name | Expression ratio Tol/CR |
|---|---|---|
| gi:20988817 - granzyme b (granzyme 2, cytotoxic t-lymphocyte-associated serine esterase 1) | mwghum40K-A04057 | 3,701538723 |
| gi:21693024 - zinc finger protein 37a; znf37a | mwghum40K-B41852 | 3,702106784 |
| gi:20538902 - similar to dj228h13.1 (similar to ribosomal protein l21e); loc148953 | mwghum40K-A41890 | 3,702475712 |
| gi:17463274 - hypothetical protein xp_069155; loc135048 | mwghum40K-B20246 | 3,710398607 |
| gi:14715065 - similar to hypothetical protein flj20008; kiaa1839 protein | mwghum40K-A31111 | 3,712412118 |
| gi:14289174-pex1pg843d; pex1g843d | mwghum40K-A02756 | 3,740677799 |
| gi:8317212 - histone acetyltransferase; mof | mwghum40K-B36960 | 3,758076607 |
| gi:23273757 - similar to serine/threonine kinase 17a (apoptosis-inducing) | mwghum40K-A09767 | 3,760413107 |
| gi:20809471 - hypothetical protein dkfzp586g0123 | mwghum40K-A12028 | 3,777595336 |
| gi:18860922 - iglc7 | mwghum40K-B43669 | 3,781190211 |
| gi:19743567 - igkv1d-8 | mwghum40K-B43562 | 3,787450736 |
| gi:21552771 - williams-beuren syndrome critical region protein 20 copy c; wbscr20c | mwghum40K-B38510 | 3,788063053 |
| gi:22050856 - hypothetical protein xp_173223; loc254756 | mwghum40K-842617 | 3,799068754 |
| gi:7243140 - kiaa1380 protein; kiaa1380 | mwghum40K-B19316 | 3,817057151 |
| gi:7688974 - uncharacterized bone marrow protein bm040 | mwghum40K-A02262 | 3,82448161 |
| gi:6005837 - polymerase (dna directed), gamma 2, accessory subunit; polg2 | mwghum40K-A09697 | 3,847835831 |
| gi:22047774 - similar to multifunctional protein ade2; loc206649 | mwghum40K-A43017 | 3,852299565 |
| gi:18546573 - similar to evidence: nas~putative~unclassifiable; loc127987 | mwghum40K-B16841 | 3,856939989 |
| gi:15929788 - suppression of tumorigenicity 13 (colon carcinoma) (hsp70-interacting protein) | mwghum40K-A10607 | 3,875925927 |
| gi:21751858 - cdna clone unnamed protein product. | mwghum40K-B38281 | 3,878056574 |
| gi:18027735 - unknown; pp11662 | mwghum40K-B41706 | 3,886720909 |
| gi:12804704 - ribosomal protein 144 | mwghum40K-B39440 | 3,887623763 |
| gi:3360463 - cytosolic aspartate aminotransferase | mwghum40K-A00653 | 3,906731022 |
| gi:21203150 - wins1 protein with drosophila lines (lin) homologous domain; wins1 | mwghum40K-B35326 | 3,965568534 |
| gi:7023151 - cdna clone unnamed protein product. | mwghum40K-B39390 | 3,969137953 |
| gi:21450921 - ighd | mwghum40K-B43573 | 4,011040093 |
| gi:7022409 - cdna clone unnamed protein product. | mwghum40K-A00667 | 4,017484733 |
| gi:263051 - membrane immunoglobulin beta chain; ig-beta/b29 | mwghum40K-A09126 | 4,033955485 |
| gi:22044729 - similar to lipase/acylhydrolase with gdsl-like motif containing protein-data source:pfam, source key:pf00657, evidence:iss~putative; loc129644 | mwghum40K-B14076 | 4,060273851 |

(continued)

| Gene accession number (GenBank) and description | Oligonucleotide name | Expression ratio Tol/CR |
|---|---|---|
| gi:12655082 - ribosomal protein s27a | mwghum40K-B41058 | 4,06622567 |
| gi:6581094 - transposase-like protein | mwghum40K-A03906 | 4,07573995 |
| gi:14010613 - methylmalonyl-coa epimerase | mwghum40K-A12449 | 4,07903176 |
| gi:559712 - kiaa0073 | mwghum40K-A14480 | 4,088796576 |
| gi:18543691 - hypothetical protein xp_105097; loc159156 | mwghum40K-B29248 | 4,098548223 |
| gi:18041651 - immunoglobulin kappa light chain variable region; igkv | mwghum40K-B40623 | 4,124292284 |
| gi:2804283 - cd94; cd94 alt | mwghum40K-A01463 | 4,159118944 |
| gi:2865465 - heat shock protein 75; hsp75 | mwghum40K-A09157 | 4,166799757 |
| gi:17933030 - ribosomal protein l30; rpl30 | mwghum40K-A19584 | 4,228101091 |
| gi:11640583 - mstp038 | mwghum40K-B14863 | 4,236244289 |
| gi:18580688 - similar to eukaryotic translation elongation factor 1 alpha 2; loc160693 | mwghum40K-B13988 | 4,241405631 |
| gi:14349352 - unknown (protein for mgc:14860) | mwghum40K-B42969 | 4,244724139 |
| gi:16553788 - cdna clone unnamed protein product. | mwghum40K-B42541 | 4,306527172 |
| gi:22042081 - similar to 60s ribosomal protein 17a (surfeit locus protein 3); loc257223 | mwghum40K-B42703 | 4,321207818 |
| gi:17932953 - ribosomal protein 131; rpl31 | mwghum40K-A42416 | 4,32535018 |
| gi:22051589 - hypothetical protein xp_085862; loc147727 | mwghum40K-B23620 | 4,344761363 |
| gi:18147104 - n27c7-4 | mwghum40K-B39728 | 4,35707073 |
| gi:5419714 - immunoglobulin light chain variable region; iglv | mwghum40K-B39513 | 4,451942254 |
| gi:17511929 - similar to wemer helicase interacting protein | mwghum40K-A06854 | 4,511622873 |
| gi:19909963 - skp2-like protein type beta; skp2-beta | mwghum40K-A16706 | 4,522162577 |
| gi:6103646 - f-box protein fbx9 | mwghum40K-B40496 | 4,544030699 |
| gi:23242916 - similar to granzyme k (serine protease, granzyme 3; tryptase ii) | mwghum40K-A07584 | 4,552800769 |
| gi:12001971 - my016 protein | mwghum40K-B34428 | 4,596380013 |
| gi:1235901 - frap-related protein; frp1 | mwghum40K-A05958 | 4,606949739 |
| gi:3668140 - pbk1 protein; pbk1 | mwghum40K-B17070 | 4,62480591 |
| gi:13366089 - ribosomal protein 17a; rp-17a | mwghum40K-A41281 | 4,656897223 |
| gi:16307226 - similar to riken cdna 1810055d05 gene | mwghum40K-B38876 | 4,772088935 |
| gi:2979600 - wugsc:h_dj0844f09.1 | mwghum40K-A40196 | 4,77523023 |
| gi:15012076 - similar to absent in melanoma 2 | mwghum40K-A15454 | 4,777925088 |
| gi:23271050 - unknown (protein for mgc:29603) | mwghum40K-A07282 | 4,818657746 |
| gi:10437386 - homo sapiens cdna: flj21308 fis, clone col02131; unnamed protein product. | mwghum40K-A02237 | 4,841262361 |
| gi:7020677 - cdna done unnamed protein product. | mwghum40K-B34056 | 4,871249521 |
| gi:16303629 - pp2a b56 gamma 1 | mwghum40K-B37873 | 4,980340639 |

(continued)

| Gene accession number (GenBank) and description | Oligonucleotide name | Expression ratio Tol/CR |
|---|---|---|
| gi:19110391 - pred16 protein; pred16 | mwghum40K-B43358 | 4,985068579 |
| gi:15082318 - unknown (protein for mgc:19920) | mwghum40K-B40521 | 4,999090084 |
| gi:13560103 - ba261n11.4.1 (klaa1510, isoform 1); ba261n11.4 | mwghum40K-B38186 | 5,042105641 |
| gi:5050949 - dj228h13.1 (similar to ribosomal protein 121e); dj228h13.1 | mwghum40K-A41684 | 5,043615944 |
| human positive control; L25899 gi:414586 - ribosomal protein l10 | mwghumcontrol11009- r2 | 5,157987898 |
| gi:16551142 - fksg19; fksg19 | mwghum40K-B34113 | 5,199863564 |
| gi:14042650 - cdna done unnamed protein product. | mwghum40K-B34706 | 5,428585203 |
| gi:14336687 - 60s ribosomal protein l23a like; rpl23al | mwghum40K-A40629 | 5,527666331 |
| gi:14327996 - unknown (protein for mgc:16362) | mwghum40K-B13027 | 5,534235926 |
| gi:21756473 - cdna clone unnamed protein product. | mwghum40K-B36364 | 5,572672719 |
| gi:14714581 - unknown (protein for mgc:15501) | mwghum40K-B15686 | 5,575058239 |
| gi:19683947 - similar to atp binding protein associated with cell differentiation | mwghum40K-B36128 | 5,688871368 |
| gi:23271260 - similar to pr domain containing 1, with znf domain | mwghum40K-B39180 | 5,721471408 |
| gi:10436784 - cdna done highly similar to mus musculus hematopoietic zinc finger protein mrna; unnamed protein product. | mwghum40K-B31498 | 5,957069561 |
| gi:12653706 - hypothetical protein | mwghum40K-A06415 | 6,184481544 |
| gi:21752198 - cdna clone unnamed protein product. | mwghum40K-B40044 | 6,409604673 |
| gi:181913 - dna-binding protein | mwghum40K-B37864 | 6,530245291 |
| gi:5453550 - cytosolic ovarian carcinoma antigen 1; cova1 | mwghum40K-A19113 | 7,068754353 |
| gi:5456958 - protocadherin gamma b2; pcdh-gamma-b2 | mwghum40K-A09293 | 11,78138569 |
| gi:22044443 - similar to riken cdna 2010110k16; riken cdna 2010312p05 gene; loc152100 | mwghum40K-B17647 | 12,82123209 |

In certain embodiments, the expression profile may further comprise at least one gene from Table 2. Preferably, the expression profile may comprise at least 1, at least 2, at least 3, at least 4, at least 5, at least 8, at least 10, at least 15, at least 20, at least 25, at least 30, or all genes from Table 2.

The following Table 2 comprises 38 additional genes that were determined by the inventors to display significantly different expression levels between kidney graft tolerant subjects as defined above (Tol) and kidney transplanted subjects in chronic rejection (CR).

Table 2. 38 additional genes differentially expressed between kidney transplanted subjects that are tolerant (Tol) or in chronic rejection (CR).

| Gene accession number (GenBank) and description | Oligonucleotide name | Expression ratio Tol/CR |
|---|---|---|
| gi:22137760 - phospholipase c-like 2 | mwghum40K-B38870 | -4,461767971 |
| gi:10835150 - solute carrier family 11 (proton-coupled divalent metal ion transporters), member 1; slc11a1 | mwghum40K-A03592 | -3,750848822 |
| gi:10835060 - inhibitor of dna binding 3, dominant negative helix-loop-helix protein; id3 | mwghum40K-A01988 | -2,980403255 |

(continued)

| Gene accession number (GenBank) and description | Oligonucleotide name | Expression ratio Tol/CR |
|---|---|---|
| gi:12803090 - cd63 antigen (melanoma 1 antigen) | mwghum40K-A01570 | -2,774109375 |
| gi:13543535 - ecotropic viral integration site 2b | mwghum40K-A06765 | -2,043057466 |
| gi:4505856 - pleiomorphic adenoma gene-like 1 isoform 1; plagl1 | mwghum40K-A05999 | -1,863821177 |
| gi:18490272 - dual specificity phosphatase 1 | mwghum40K-A09573 | -1,806051907 |
| gi:5263001 - dj164f3.4 (bruton agammaglobulinemia tyrosine kinase); btk | mwghum40K-A35460 | -1,769076236 |
| gi:435598 - interleukin 1-beta convertase; il1bce | mwghum40K-A06096 | -1,755287579 |
| gi:5454011 - ring finger protein 3; mf3 | mwghum40K-A10138 | -1,645215839 |
| gi:6468774 - protein translation initiation factor 2c2; eif2c2 | mwghum40K-A36858 | -1,606505506 |
| gi:4506750 - restin (reed-steinberg cell-expressed intermediate filament-associated protein); rsn | mwghum40K-A04890 | -1,489393697 |
| gi:17389476 - similar to pleckstrin homology, sec7 and coiled/coil domains 4 | mwghum40K-A36644 | -1,436953848 |
| gi:17986253 - sp110 nuclear body protein, isoform c; sp110 | mwghum40K-A01745 | -1,416856208 |
| gi:4557874 - dihydropyrimidine dehydrogenase; dpyd | mwghum40K-A00779 | -1,291235355 |
| gi:4758751 - baculoviral iap repeat-containing 1; birc1 | mwghum40K-A04764 | -1,1861284 |
| gi:22046239 - similar to neutrophil cytosolic factor 1 (47kd, chronic granulomatous disease, autosomal 1); ncf1 | mwghum40K-A39023 | -1,104625992 |
| gi:15929529 - fc fragment of ige, high affinity i, receptor for; alpha polypeptide | mwghum40K-A02083 | 1,202355712 |
| gi:4505356 - nadh dehydrogenase (ubiquinone) 1 alpha subcomplex, 4 (9kd, mlrq); ndufa4 | mwghum40K-A39699 | 1,206373182 |
| gi:14327951 - rho gdp dissociation inhibitor (gdi) beta | mwghum40K-A00484 | 1,28076071 |
| gi:5174726 - chaperonin containing tcp1, subunit 3 (gamma); cct3 | mwghum40K-A00632 | 1,30370066 |
| gi:14589887 - cadherin 1, type 1 preproprotein; cdh1 | mwghum40K-A08852 | 1,783062394 |
| gi:21536313 - ret proto-oncogene isoform b; ret | mwghum40K-A03023 | 1,811124453 |
| gi:11121380 - ba421h8.4.2 (clathrin, light polypeptide (lca)); clta | mwghum40K-A04600 | 1,910743697 |
| gi:4507126 - small nuclear ribonucleoprotein polypeptide c; snrpc | mwghum40K-A02770 | 2,00949673 |
| gi:16924322 - replication factor c (activator 1) 4 (37kd) | mwghum40K-A07827 | 2,011149889 |
| gi:12654886 - histidine triad nucleotide-binding protein | mwghum40K-A05885 | 2,069102874 |
| gi:15088549 - proapoptotic caspase adaptor protein | mwghum40K-A17881 | 2,230034551 |
| gi:1203968 - filamin; fln | mwghum40K-A19601 | 2,234253841 |
| gi:11038671 - cd79a antigen, isoform 2 precursor; cd79a | mwghum40K-A09786 | 2,512467355 |
| gi:21902515 - proliferating cell nuclear antigen; pcna | mwghum40K-A07774 | 2,629339122 |
| gi:3153208 - calumein; calu | mwghum40K-A31686 | 2,907912027 |
| gi:18699568 - macrophage migration inhibitory factor; mif | mwghum40K-A05478 | 3,077358724 |

(continued)

| Gene accession number (GenBank) and description | Oligonucleotide name | Expression ratio Tol/CR |
|---|---|---|
| gi:22208950 - ankyrin repeat and socs box-containing protein 3 isoform b; asb3 | mwghum40K-A05294 | 3,201230331 |
| gi:2213931 - 26s proteasome regulatory subunit; sug2 | mwghum40K-A01842 | 3,471916901 |
| gi:5032004 - protein tyrosine phosphatase, receptor type, c-associated protein; ptprcap | mwghum40K-A03948 | 3,531892306 |
| gi:22049940 - similar to dynein, cytoplasmic, light peptide; loc255708 | mwghum40K-B42942 | 3,934790681 |
| gi:3859866 - cytochrome c oxidase subunit vic; cox6c | mwghum40K-A40363 | 4,257734604 |

In a particular embodiment, the determination of the presence or absence of a graft tolerant phenotype is carried out by comparison of the obtained expression profile with at least one reference expression profile.

A "reference expression profile" is a predetermined expression profile, obtained from a pooled biological sample constituted of sample(s) from at least one subject with a known particular graft tolerance state. In particular embodiments, the reference expression profile may have been obtained from pooled biological samples respectively constituted of sample(s) from at least one graft tolerant subject ("tolerant reference expression profile"), or sample(s) from at least one graft non-tolerant subject ("non-tolerant reference expression profile"), in particular subjects in chronic rejection.

Preferably, at least one reference expression profile is a tolerant reference expression profile. Alternatively, at least one reference expression profile may be a non-tolerant reference expression profile. More preferably, the determination of the presence or absence of a graft tolerant phenotype is carried out by comparison with both tolerant and non-tolerant reference expression profiles.

The comparison of a tested subject expression profile with a reference expression profile may be performed using PAM (predictive analysis of microarrays) statistical method. A non supervised PAM 2 classes statistical analysis is performed. For comparison with a tolerant reference expression profile, a cross validation (CV) probability is calculated ($CV_{tol}$), which represents the probability that the tested subject is tolerant. For comparison with a chronic rejection (non-tolerant) reference expression profile, a CV probability is calculated ($CV_{CR}$), which represents the probability that the tested subject is undergoing chronic rejection. The diagnosis is then performed based on the $CV_{tol}$ and/or the $CV_{CR}$ probabilities. Preferably, a tolerant subject has a $CV_{tol}$ probability of at least 0.80, at least 0.85, more preferably at least 0.90, at least 0.95, at least 0.97, at least 0.98, at least 0.99, or even 1.00, and has a $CV_{CR}$ probability of at most 0.20, at most 0.15, at most 0.10, at most 0.05, at most 0.03, at most 0.02, at most 0.01, or even 0.00.

The expression profile may be determined by any technology known by a man skilled in the art. In particular, each gene expression level may be measured at the nucleic and/or proteic level. In a preferred embodiment, the expression profile is determined by measuring the amount of nucleic acid transcripts of each gene. In another embodiment, the expression profile is determined by measuring the amount of each gene corresponding protein.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a preferred embodiment, the expression profile is determined using quantitative PCR.

In another preferred embodiment, the expression profile is determined by the use of a nucleic microarray.

According to the invention, a "nucleic microarray" consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art, such as those described in patents or patent applications US 5,143,854; US 5,288,644; US 5,324,633; US 5,432,049; US 5,470,710; US 5,492,806; US 5,503,980; US 5,510,270; US 5,525,464; US 5,547,839; US 5,580,732; US 5,661,028; US 5,800,992; WO 95/21265; WO 96/31622; WO 97/10365; WO 97/27317; EP 373 203; and EP 785 280; the disclosures of which are herein incorporated by reference.

In a preferred embodiment, the nucleic microarray is an oligonucleotide microarray comprising at least one oligonucleotide specific for a gene from Table 1. Preferably, the oligonucleotides are about 50 bases in length.

The oligonucleotide microarray may further comprise at least one oligonucleotide specific for a gene from Table 2. Preferably, the oligonucleotides are about 50 bases in length.

Suitable microarray oligonucleotides specific for any gene from Table 1 or Table 2 may be designed, based on the genomic sequence of each gene (see Tables 1 and 2 Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

The invention is also directed to a method for the in vitro diagnosis of a graft tolerant phenotype from at least one biological sample of a grafted subject, comprising :

(a) measuring standard biological parameters specific for said subject grafted organ type,
(b) determining an expression profile comprising at least one gene from Table 1,
(c) determining the presence or absence of a graft tolerant phenotype from the results of steps (a) and (b).

According to the invention, "standard biological parameters specific for said subject grafted organ type" means biological parameters that are usually used by clinicians to monitor the stability of grafted subjects status and to detect graft rejection. These standard biological parameters specific for said subject grafted organ type usually comprise serum or plasma concentrations of particular proteins, which vary depending on the grafted organ type. However, these standard biological parameters specific for said subject grafted organ type are, for each organ type, well known of those skilled in the art.

For instance, standard biological parameters specific for kidney include serum or plasma urea and creatinine concentrations. In a healthy subject, the serum creatinine concentration is usually comprised between 40 to 80 $\mu$mol/L for a woman and 60 to 100 $\mu$mol/L for a man, and the serum urea concentration between 4 to 7 mmol/L.

For instance, for liver transplantation, standard biological parameters include serum or plasma concentrations of gamma glutamyl transpeptidase (GGT), aspartate aminotransferase (AST), alanine aminotransferase (ALT), lactate dehydrogenase (LDH), and bilirubin (total or conjugated).

These standard biological parameters have the advantage of being easily measurable from a blood sample, but are not sufficient to establish a precise graft tolerant or non-tolerant diagnosis, and are also not enough sensitive to allow an early chronic rejection diagnosis. However, when combined with the determination of an expression profile according to the present invention, the resulting method according to the invention makes it possible to detect graft tolerant subject whose immunosuppressive treatment could be progressively decreased, as well as apparently stable patients (relative to their biological parameters) who are actually on the verge of chronic rejection.

Other parameters that cannot be used alone for a diagnosis but that have been described as displaying significantly different values between tolerant grafted subjects and subjects in chronic rejection may also be used to refine and/or confirm the diagnosis according to the above described method according to the invention combining the measure of standard biological parameters and the determination of an expression profile. These optional supplementary parameters include the percentage of CD4$^+$ CD25$^+$ T cells in peripheral blood lymphocytes (Brouard et al. 2005. Am J Transplant. 5(2):330-40), as well as parameters relative to the qualitative and quantitative analysis of the T cell repertoire (Brouard et al. 2005. Am J Transplant. 5(2):330-40), such as the T cell repertoire oligoclonality and the level of TCR transcripts.

The percentage of CD4$^+$ CD25$^+$ T cells in peripheral blood lymphocytes may be determined by any technology known in the art, in particular by flow cytometry using labelled antibodies specific for the CD4 and CD25 molecules. Preferably, the percentage of CD4$^+$ CD25$^+$ T cells in peripheral blood lymphocytes of a tolerant subject is not statistically different from that of a healthy volunteer, whereas it is significantly lower ($p < 0.05$) in a non-tolerant grafted subject.

The T cell repertoire oligoclonality may be determined by any technology enabling to quantify the alteration of a subject T cell repertoire diversity compared to a control healthy subject. Usually, said alteration of a subject T cell repertoire diversity compared to a control healthy subject is determined by quantifying the alteration of T cell receptors (TCR) complementary determining region 3 (CDR3) size distributions. Indeed, in a control healthy subject, such a TCR CDR3 size distribution displays a Gaussian form, which may be altered in the presence of clonal expansions due to immune response, or when the T cell repertoire diversity is limited and reaches oligoclonality.

The level of TCR transcripts is preferably determined independently for each V$\beta$ family by any technology known in the art. For instance, the level of TCR transcripts of a particular V$\beta$ family may be determined by calculating the ratio between these V$\beta$ transcripts and the transcripts of a control housekeeping gene, such as the HPRT gene.

Preferably, in a graft tolerant subject, a significant percentage of Vβ families display an increase in their transcript numbers compared to a normal healthy subject.

Methods to analyze T cell repertoire oligoclonality and/or the level of TCR transcripts, as well as scientific background relative to T cell repertoire, are clearly and extensively described in WO 02/084567, which is herein incorporated by reference. Preferably, a graft tolerant subject, as well as a subject in chronic rejection, displays a T cell repertoire with a significantly higher oligoclonality than a normal healthy subject.

The cytokine expression profile of T cells may be determined using any technology known in the art, including quantitative PCR and intracellular flow cytometry analysis. Preferably, the oligoclonal Vβ families of a non-tolerant grafted subject express increased levels compared to a healthy volunteer of TH1 or TH2 effector molecules, including interleukin 2 (IL-2), interleukin 8 (IL-8), interleukin 10 (IL-10), interleukin 13 (IL-13), transforming growth factor beta (TGF-β), interferon gamma (IFN-γ) and perforin, whereas oligoclonal Vβ families of a tolerant grafted subject do not express increased levels of those effector molecules compared to a healthy volunteer.

In a particular embodiment of the above described method according to the invention combining the measure of standard biological parameters and the determination of an expression profile, said method may thus further comprise a (b2) step between steps (b) and (c), which consists in studying at least one other parameter chosen in the group comprising:

- the percentage of CD4$^+$ CD25$^+$ T cells in peripheral blood lymphocytes,
- parameters relative to the T cell repertoire, such as the T cell repertoire oligoclonality and the level of TCR transcripts, and
- the cytokine expression profile of T cells oligoclonal Vβ families,

and wherein step (c) further takes into account the results of step (b2).

In a particular embodiment, step (c) is carried out by following a decision algorithm that takes into account the results of steps (a) and (b), and optionally of step (b2).

By a "decision algorithm" is meant a set of instructions allowing, for any particular steps (a), (b), and optionally (b2) results, the automated diagnosis of a graft tolerant or non-tolerant phenotype. Such an algorithm may be a list of dependant and/or independent instructions leading to a graft tolerant or non-tolerant phenotype diagnosis, or a software which, after entry of the different results, returns either a graft tolerant or non-tolerant phenotype diagnosis.

In a preferred embodiment of any above described in vitro diagnosis method according to the invention, said method is performed on a biological sample from a kidney transplanted subject. According to the invention, a "kidney transplanted subject" is a subject that was grafted with a non syngeneic, including allogenic or even xenogenic, kidney. Said kidney transplanted subject may further have been grafted with another organ of the same donor providing the kidney. In particular, said kidney transplanted subject may further have been grafted with the pancreas, and optionally a piece of duodenum, of the kidney donor.

For such a kidney transplanted subject, step (c) of a method of in vitro diagnosis taking into account both standard biological parameters specific for said subject grafted organ type and an expression profile comprising at least one gene of Table 1, and optionally at least one gene of Table 2, is preferably performed using the following decision algorithm:

(a) if the serum concentration of urea is superior to 7 mmol/L or the serum concentration of creatinine is superior to 80 μmol/L for a female subject or 100 μmol/L for a male subject, then the tested subject is diagnosed as not tolerant to his graft

(b) if the serum concentration of urea is inferior to 7 mmol/L and the serum concentration of creatinine is inferior to 80 μmol/L for a female subject or 100 μmol/L for a male subject, then the diagnosis is performed by determining an expression profile according to the invention.

The invention further concerns a kit for the in vitro diagnosis of a graft tolerant phenotype, comprising :

(a) at least one reagent for the determination of an expression profile comprising at least one gene from Table 1, and
(b) instructions for the determination of the presence or absence of a graft tolerant phenotype.

Such a kit for the in vitro diagnosis of a graft tolerant phenotype may further comprise at least one reagent for the determination of an expression profile also comprising at least one gene from Table 2.

In another embodiment, a kit for the in vitro diagnosis of a graft tolerant phenotype according to the invention may further comprise at least one reagent for the measure of standard biological parameters specific for at least one grafted organ type. Such as kit may still further comprise at least one reagent chosen in the group comprising reagents for the measure of the percentage of CD4$^+$ CD25$^+$ T cells in peripheral blood lymphocytes, reagents for the analysis of T cell repertoire, and reagents for the measure of the cytokine expression profile of T cells oligoclonal Vβ families.

In any kit for the in vitro diagnosis of a graft tolerant phenotype according to the invention, the reagent(s) for the determination of an expression profile comprising at least one gene from Table 1, and optionally at least one gene from Table 2, preferably include specific amplification primers and/or a nucleic microarray.

In addition, the instructions for the determination of the presence or absence of a graft tolerant phenotype preferably include at least one reference expression profile. In a preferred embodiment, at least one reference expression profile is a graft tolerant expression profile. Alternatively, at least one reference expression profile may be a graft non-tolerant expression profile. More preferably, the determination of the level of graft tolerance is carried out by comparison with both graft tolerant and graft non-tolerant expression profiles.

In a particular embodiment, the instructions for the determination of the presence or absence of a graft tolerant phenotype may further include a decision algorithm, as defined above. In the case of a kidney grafted subject, the instructions for the determination of the presence or absence of a graft tolerant phenotype may be constituted of the decision algorithm described above.

The invention is also directed to an oligonucleotide microarray comprising a plurality of oligonucleotides specific for a gene associated with a graft tolerant phenotype, wherein said oligonucleotides comprise at least one oligonucleotide specific for a gene from Table 1. Preferably, said oligonucleotide microarray allows for the detection of at least 1, at least 2, at least 3, at least 4, at least 5, at least 8, at least 10, at least 15, at least 20, at least 30, at least 40, at least 50, at least 75, at least 100, at least 200, at least 300, or all genes from Table 1.

In a particular embodiment, said oligonucleotide microarray may further comprise at least one oligonucleotide specific for a gene from Table 2. Preferably, said oligonucleotide microarray comprises oligonucleotides specific for at least 1, at least 2, at least 3, at least 4, at least 5, at least 8, at least 10, at least 15, at least 20, at least 25, at least 30, or all genes from Table 2.

The invention is also drawn to a method of treatment of a grafted subject, comprising:

(a) determining from a subject biological sample the presence or absence of a graft tolerant phenotype using a method according to the invention, and
(b) adapting the immunosuppressive treatment in function of the result of step (a).

Having generally described this invention, a further understanding of characteristics and advantages of the invention can be obtained by reference to certain specific examples and figures which are provided herein for purposes of illustration only and are not intended to be limiting unless otherwise specified.

## DESCRIPTION OF THE DRAWINGS

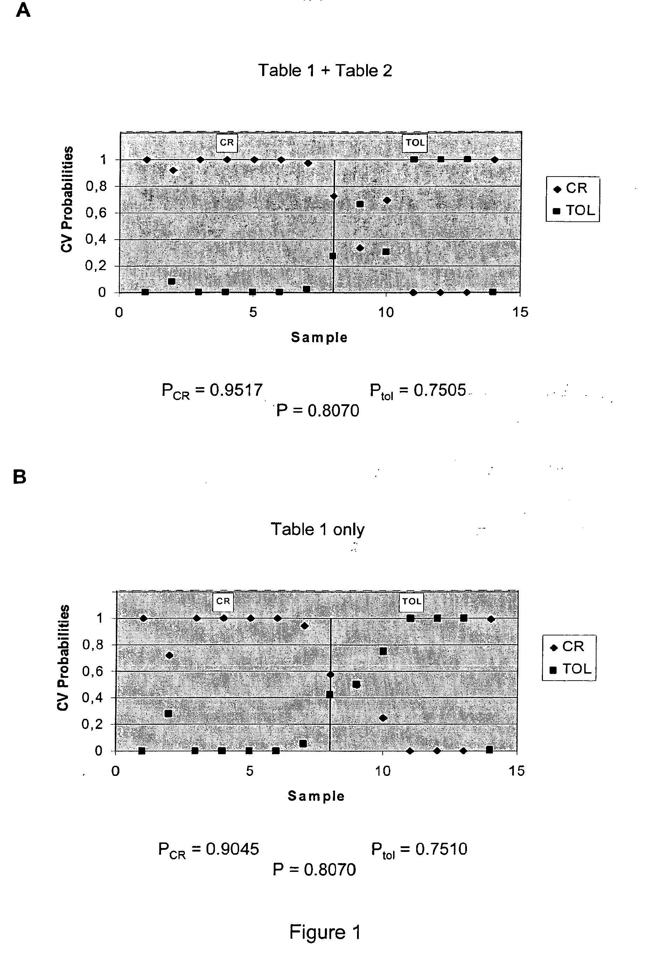

Figure 1. Validation of the diagnosis predictive value of dedicated microarrays comprising oligonucleotides specific for Tables 1 and 2 genes (A) or Table 1 only genes (B). The samples of individual tested patients are plotted in abscissa, the chronic rejection group (samples n°1 to 8) on the left, the tolerant group (samples n°9 to 14) on the right (see separation bar). In ordinates are plotted both the chronic rejection (black rhomb) and tolerant (gray square) CV probabilities for each patient sample. Probabilities to identify a tolerant patient ($P_{tol}$) or a chronic rejection patient ($P_{CR}$), as well as the probability to correctly identify the phenotype of an individual subject (P), are also stated.

## EXAMPLE 1

Identification of differentially expressed genes between tolerant and non-tolerant patients (chronic rejection)

1.1 Patients selection

All patients were more than 18 years old and had been kidney transplanted for more than 5 years.

Tolerant patients ("Tol group"; 52.29 $\pm$ 17.19 years old; n=7) had stable renal function and had taken no immunosuppressive drug treatment for at least 3 years.

Non-tolerant patients in chronic rejection ("CR group"; 50.45 $\pm$ 14.45 years old; n=11) are defined thanks to histological chronic rejection lesions, with or without allograft glomerular disease and renal function degradation.

1.2 Preparation, amplification, and staining of messenger RNA (mRNA)

A patient blood sample is diluted to ½ with PBS and deposited on Ficoll gradient. Extracted RNA quantity and quality are tested on Agilent 2100 Bioanalyser®.

The amplification and staining of mRNA is carried out using "Amino Allyl MessageAmp™ aRNA" kit (Ambion®; Austin, Texas).

This method consist in the retrotranscription of mRNA with an oligo(dT) primer comprising a T7 promoter ("T7 oligo(dT) primer"), the in vitro transcription of resulting DNA with T7 RNA polymerase, generating anti-sens RNA copies of each mRNA. This kit allows for the incorporation of modified nucleotides, 5-(3-aminoallyl)-UTP (aaUTP) in amplified RNA during in vitro transcription. aaUTP contains a primary amino group in uracile C5 position which is chemically linked to a fluorophore. Once purified, stained aRNA are used for hybridization on DNA microarrays. All words under quotation marks refer to reagents provided in the kit.

A mixture of 5 μg of total RNA, 1 μg of T7 oligo(dT) primer and RNase free water qsp to 12 μL is incubated for 10 minutes at 70°C. 8 μL of a mixture consisting of 2 μL of "10X First Strand Buffer", 1 μL of "Ribonuclease inhibitor", 4 μL of "dNTP mix" and 1 μL of "Reverse Transcriptase", are added to each sample which is then incubated for 2 hours at 42°C.

63 μL of nuclease free water, 10 μL of "10X Second Strand Buffer", 4 μL od "dNTP Mix", 2 μL of DNA polymerase and 1 μL of RNase H are added to each sample, which is further incubated for 2 hours at 16°C. 250 μL of "cDNA binding Buffer" are added to each sample, which is then deposited on a purification column and centrifugated for 1 minute at 10000 g. The liquid is removed and 500 μL of "cDNA Wash Buffer" are added before further centrifugation for 1 minute at 10000 g. The column is then placed in a new receiving tube and 9 μL of RNase free water (pre-heated to 50°C) are deposited in the centre of the column for 2 minutes, and then the column is centrifugated for 1.5 minute at 10000 g, thus eluting cDNA.

To 14 μL of cDNA are added 3 μL of "aaUTP Solution" (50 mM), 12 μL of "ATP, CTP, GTP Mix" (25 mM), 3 μL of "UTP Solution" (50 mM), 4 μL of "T7 10X Reaction Buffer" and 4 μL of "T7 Enzyme Mix". The reaction mixture is incubated for 6 to 14 hours at 37°C. Contaminating cDNA is removed by the addition of 2 μL of DNase I for 30 minutes at 37°C. 58 μL of nuclease free water, 350 μL of "aRNA Binding Buffer" and 250 μL of ethanol are added to each sample, which is then deposited on a column and centrifugated for 1 minute at 10000 g. The column is then placed in a new tube and 650 μL of "aRNA Wash Buffer" are deposited onto the column, which is then twice centrifugated for 1 minute at 10000 g and transferred into a new tube. 50 μL of nuclease free water (pre-heated to 50°C) is then deposited onto the column centre. After 2 minutes of incubation, the column is centrifugated for 1,5 minute at 10000 g.

Resulting aRNA is dosed thanks to Agilent 2100 Bioanalyser®, as previously. 5 to 20 μg of aRNA are concentrated to a final volume of 7 μL. 9 μL of "Coupling Buffer", 4 μL of "ester dye Cyanine 3" (Cy3) and 4 μL of "ester dye Cyanine 5" (Cy5) in DMSO (Amersham Biosciences; Orsay, France) are added to each sample. After 30 minutes incubation, 4.5 μL of "Hydroxylamine 4M" are added for 15 minutes. Then 500 μL of "Capture Buffer" are added to each sample, which is then deposited on a GFX column. After a 30 seconds centrifugation at 13000 rpm, columns are washed with 600 μL of "Wash Buffer" and centrifugated for 30 seconds at 13000 rpm. The washing step is repeated twice, then 60 μL of nuclease free water preheated to 70°C are deposited onto the column centre. After 3 minutes incubation, columns are centrifugated for 1 minute at 13000 rpm.

The fluorophore incorporation is measured using spectrophotometry, and is expressed as the number of fluorophore molecules/1000 nucleotides, according to the following formula:

$$\frac{\text{Number of dyes}}{1000 \text{ nucleotides}} = \frac{\text{dye}.9010 \text{ M}^{-1} \text{ cm}^{-1}.1000}{\text{A260}.\text{Dye extinction coefficient}}$$

The coupling reaction is satisfactory if 20 to 50 fluorophore molecules/1000 nucleotides are incorporated.

1.3 Hybridization of aRNA on microarrays

Slides preparation: Pan-genomic microarrays (MWG Biotech; Courtaboeuf, France) are incubated for 15 minutes at 50°C in a solution comprising ethanolamine (50 mM; Sigma; St Quenyin Fallavier, France), Tris-base 100 mM pH9, SDS 0.1% (BIO-RAD; Marne-la-Coquette, 5 times going up and down for each wash), and then incubated for 15 minutes at 50°C in SSC 4X (Invitrogen; Cergy Pontoise, France), 0.1% SDS. After 1 hour prehybridization at 42°C in 150 mL of SSC 3.5X, SDS 0.3%, BSA 1% (Sigma; St Quenyin Fallavier, France), microarrays are washed in 5 water baths at room temperature, and further centrifugated for 3 minutes at 500 rpm at room temperature.

Hybridization: aRNA are denaturated for 2 minutes at 95°C and then incubated for 30 minutes at 37°C. RNA are placed on the spotted zone and a lamellae is put on the microarray in such a way to prevent bubbles formation. The ensemble is placed in an hybridization chamber, 20 μL of SSC 3X are put in the chamber tank, and 3 droplets of SSC 3X are deposited on the microarray, near from the lamellae. The hybridization chamber is hermetically closed and immersed overnight in obscurity in a closed water bath at 42°C. Microarrays are then immersed in a SSC 2X, SDS 0.1% buffer, and further transferred in a SSC 1X buffer and stirred for 2 minutes. After a 2 minutes wash in SSC 0.2X, they are centrifuged at room temperature for 3 minutes at 500 rpm. They are then treated with 350 μL of Dyesaver (Genisphere) and dried 3 minutes in open air. Hybridized microarrays must be protected from light and dust and stored at room temperature.

1.4 Data acquisition and analysis

Microarrays reading is performed using a ScanArray® scanner (GSI Lumonics) at two distincts wavelengths: 633 nm for Cyanine 5 excitation and 532 nm for Cyanine 3 excitation. Resulting images are analyzed using GenePix Pro 5.1® (Axon Instruments, Inc) software. This software allows, via customizable grids, to ascribe each spot identity and to determine if each spot is or not valid in function of its homogeneity and intensity. A manual proofreading permits to validate and/or correct the software analysis. Raw results are then further analyzed using a macro developed on IFR26 website: "MADSCAN" (MicroArray Data Suites of Computer Analysis), accessible on http://www.madtools.org/. This macro is useful for data filtration (signal ratio values, background noise value, saturation value...), data normalization by non linear regression, outliers detection, and data integration. The obtained results are then analyzed by a supervised technology (SAM, "Significance Analysis of Microarray").

Genes expressed on at least 2 microarrays out of 4 for which a "one class" SAM analysis has been performed were selected. This analysis allows for the determination of genes that are significantly differentially expressed between the Tol and the CR group (q<5%).

1.5 Results: differentially expressed genes between the Tol and CR groups

Using the above described protocol, the inventors identified 310 genes that are significantly differentially expressed between kidney graft tolerant subjects and kidney transplanted subjects in chronic rejection. These genes are listed in above inserted Table 1 and Table 2.

**EXAMPLE 2**

Validation of the predictive value of an expression profile comprising genes listed in Table 1, or Table 1 and Table 2

2.1 Principle and methods

The genes listed in Tables 1 and 2 were identified as differentially expressed between tolerant patients and patients in chronic rejection using pangenomic microarrays (around 40000 genes) and pooled tolerant and chronic rejection samples, respectively constituted of several tolerant patients samples and several samples from patients in chronic rejection.

To assess the predictive value of a diagnosis performed using an expression profile comprising genes from Table 1, or Table 1 and Table 2, a validation step was performed with dedicated oligonucleotide microarrays comprising oligonucleotides specific for genes of Table 1 and Table 2, on individual patients with a phenotype of tolerance (6) or chronic rejection (8).

This validation step allows to determine the predictive value of such an expression profile in an individual, which is crucial for diagnosis since each patient will be tested autonomously.

The validation process uses PAM (predictive analysis of microarrays) statistical method. Only genes for which data is available for at least 50% of tolerant and under chronic rejection patients are taken into account in the analysis. A non supervised PAM 2 classes statistical analysis is performed.

2.2 Results

All individual patients included in the validation step were analyzed without knowing to which group they belong (tolerant or chronic rejection).

For each patient, two cross validation (CV) probabilities (comprised between 0 and 1) were calculated, corresponding respectively to the probability, calculated based on their expression profile on the dedicated microarray, that said patient belong to either the tolerant group, or the group of patients in chronic rejection.

The CV probabilities of the 6 tolerant patients and the 8 patients in chronic rejection are plotted in Figure 1, either with Table 1 and Table 2 genes (Figure 1A) or with only Table 1 genes (Figure 1B).

Moreover, the mean probability to accurately identify a tolerant subject within the tolerant group ($P_{tol}$) and a

patient in chronic rejection in the chronic rejection group ($P_{CR}$), as well as the mean value of these two probabilities (P), were calculated and are displayed in Figure 1.

Results, which are summarized in Figure 1, show that the dedicated microarrays have a high predictive diagnostic value, since the probability to identify a tolerant patient ($P_{tol}$) is superior to 0.75, with or without Table 2 genes, while the probability to identify a patient in chronic rejection ($P_{CR}$) is superior to 0.90, with or without Table 2 genes.

Overall, the probability to correctly identify the phenotype of an individual subject (P) is superior to 0.80, with or without Table 2 genes.

**Claims**

1. Method for the in vitro diagnosis of a graft tolerant phenotype, comprising:

    (a) determining from a grafted subject biological sample an expression profile comprising at least one gene from Table 1, and
    (b) determining the presence or absence of a graft tolerant phenotype from said expression profile.

2. The method of claim 1, wherein the expression profile further comprises at least one gene from Table 2.

3. The method of claims 1-2, wherein the determination of the presence or absence of a graft tolerant phenotype is carried out by comparison of the obtained expression profile with at least one reference expression profile.

4. The method of claim 3, wherein at least one reference expression profile is a tolerant expression profile.

5. The method of claim 3, wherein at least one reference expression profile is an non-tolerant expression profile.

6. The method of claim 3, wherein both a tolerant and an non-tolerant expression profile are used in step (b).

7. The method of claims 1-6, wherein the expression profile is determined by measuring the amount of nucleic acid transcripts of said gene(s).

8. The method of claim 7, wherein the expression profile is determined using quantitative PCR.

9. The method of claim 7, wherein the expression profile is determined by the use of a nucleic microarray.

10. The method of claim 9, wherein the nucleic microarray is an oligonucleotide microarray comprising at least one oligonucleotide from Table 1.

11. The method of claim 10, wherein said oligonucleotide microarray further comprises at least one oligonucleotide from Table 2.

12. A method for the in vitro diagnosis of a graft tolerant phenotype from at least one biological sample of a grafted subject, comprising :

    (a) measuring standard biological parameters specific for said subject grafted organ type,
    (b) determining an expression profile comprising at least one gene from Table 1,
    (c) determining the presence or absence of a graft tolerant phenotype from the results of steps (a) and (b).

13. The method of claim 12, further comprising a (b2) step between steps (b) and (c), which consists in studying at least one other parameter chosen in the group comprising:

    - the percentage of CD4$^+$ CD25$^+$ T cells in peripheral blood lymphocytes,
    - parameters relative to the T cell repertoire, such as the T cell repertoire oligoclonality and the level of TCR transcripts, and
    - the cytokine expression profile of T cells oligoclonal Vβ families,

    and wherein step (c) further takes into account the results of step (b2).

**14.** The method of claim 12 or claim 13, wherein step (c) is carried out by following a decision algorithm that takes into account the results of steps (a) and (b), and optionally of step(b2).

**15.** The method according to any of claims 1-14, wherein said method is performed on a biological sample from a kidney transplanted subject.

**16.** The method according to claim 14 and 15, wherein said decision algorithm is the following:

(a) if the serum concentration of urea is superior to 7 mmol/L or the serum concentration of creatinine is superior to 80 $\mu$mol/L for a female subject or 100 $\mu$mol/L for a male subject, then the tested subject is diagnosed as not tolerant to his graft
(b) if the serum concentration of urea is inferior to 7 mmol/L and the serum concentration of creatinine is inferior to 80 $\mu$mol/L for a female subject or 100 $\mu$mol/L for a male subject, then the diagnosis is performed by determining an expression profile using a method according to any of claims 1-11.

**17.** A kit for the in vitro diagnosis of a graft tolerant phenotype, comprising :

(a) at least one reagent for the determination of an expression profile comprising at least one gene from Table 1, and
(b) instructions for the determination of the presence or absence of a graft tolerant phenotype.

**18.** The kit of claim 17, wherein said expression profile further comprises least one gene from Table 2.

**19.** The kit of any of claims 17-18, further comprising at least one reagent for the measure of standard biological parameters specific for at least one grafted organ type.

**20.** The kit of any of claims 17-19, further comprising at least one reagent chosen in the group comprising reagents for the measure of the percentage of CD4$^+$ CD25$^+$ T cells in peripheral blood lymphocytes, reagents for the analysis of T cell repertoire, and reagents for the measure of the cytokine expression profile of T cells oligoclonal V$\beta$ families.

**21.** The kit of any of claims 17-20, wherein the reagent(s) for the determination of an expression profile comprising at least one gene from Table 1, and optionally at least one gene from Table 2, include specific amplification primers and/or a nucleic microarray, and the instructions for the determination of the presence or absence of a graft tolerant phenotype include at least one reference expression profile.

**22.** An oligonucleotide microarray comprising a plurality of oligonucleotides specific for a gene associated with a graft tolerant phenotype, wherein said oligonucleotides comprise at least one oligonucleotide specific for a gene from Table 1.

**23.** The oligonucleotide microarray of claim 22, further comprising at least one oligonucleotide specific for a gene from Table 2.

**A**

Table 1 + Table 2

$P_{CR} = 0.9517$  $P_{tol} = 0.7505$

$P = 0.8070$

**B**

Table 1 only

$P_{CR} = 0.9045$  $P_{tol} = 0.7510$

$P = 0.8070$

Figure 1

European Patent
Office

**EUROPEAN SEARCH REPORT**

Application Number

EP 05 29 1215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/005601 A (THE REGENTS OF THE UNIVERSITY OF MICHIGAN; CLARKE, MICHAEL, F; LIU, RU) 20 January 2005 (2005-01-20) | 17-23 | C12Q1/68 |
| A | * page 119, line 3 - line 4; table 5 * ----- | 1-16 | |
| A | SCHERER A ET AL: "EARLY PROGNOSIS OF THE DEVELOPMENT OF RENAL CHRONIC ALLOGRAFT REJECTION BY GENE EXPRESSION PROFILING OF HUMAN PROTOCOL BIOPSIES" TRANSPLANTATION, WILLIAMS AND WILKINS, BALTIMORE, MD, US, vol. 75, no. 8, 27 April 2003 (2003-04-27), pages 1323-1330, XP009045201 ISSN: 0041-1337 * the whole document * ----- | 1-23 | |

TECHNICAL FIELDS
SEARCHED    (IPC)

C12Q

~~The present search report has been drawn up for all claims~~

| Place of search | Date of completion of the search | Examiner |
|---|---|---|
| Munich | 27 March 2006 | Sprinks, M |

EPO FORM 1503 03.82 (P04C01)

European Patent

Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-23 partially

24

**European Patent
Office**

**LACK OF UNITY OF INVENTION
SHEET B**

The Search Division considers that the present European patent application does not comply with the
requirements of unity of invention and relates to several inventions or groups of inventions, namely:

Invention 1: claims 1-23 partially

       Methods of assessing graft tolerance using a polynucleotide
       corresponding to the first gene ( in Table 1, and
       subject-matter relating thereto.
                    ---

Inventions 2-272: claims 1-23 partially

       idem invention 1 for the other 271 genes in Table 1
       respectively.
                    ---

**ANNEX TO THE EUROPEAN SEARCH REPORT
ON EUROPEAN PATENT APPLICATION NO.**

EP 05 29 1215

This annex lists the patent family members relating to the patent documents cited in  the above-mentioned European search report.
The members are as contained in the European Patent Office EDP file on
The European Patent Office is in no way liable for these particulars which are merely  given for the purpose of information.

27-03-2006

| Patent document cited in search report | Publication date | Patent family member(s) | | Publication date |
|---|---|---|---|---|
| WO 2005005601    A | 20-01-2005 | AU | 2004256425 A1 | 20-01-2005 |
| | | US | 2006019256 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5143854 A
- US 5288644 A
- US 5324633 A
- US 5432049 A
- US 5470710 A
- US 5492806 A
- US 5503980 A
- US 5510270 A
- US 5525464 A
- US 5547839 A
- US 5580732 A
- US 5661028 A
- US 5800992 A
- WO 9521265 A
- WO 9631622 A
- WO 9710365 A
- WO 9727317 A
- EP 373203 A
- EP 785280 A
- WO 02084567 A

**Non-patent literature cited in the description**

- **DANTAL J. et al.** *Lancet,* 1998, vol. 351, 623-8
- **IMAN A. et al.** *Natl. Med. J. India.,* 2001, vol. 14 (2), 75-80
- **BROUARD S. et al.** *Am. J. Transplant.,* 2005, vol. 5 (2), 330-340
- **NANKIVELL et al.** *N Engl J Med.,* 2003, vol. 349 (24), 2326-33
- **BROUARD et al.** *Am J Transplant.,* 2005, vol. 5 (2), 330-40